# EUROPÄISCHE PATENTSCHRIFT

(11) **EP 2 884 880 B1**
(45) Veröffentlichungstag und Bekanntmachung des Hinweises auf die Patenterteilung: **08.06.2022**
(21) Anmeldenummer: 13763000.0
(22) Anmeldetag: 14.08.2013
(51) Int. Cl.: A61B 1/045, A61B 1/24, A61B 1/247

(54) **VERFAHREN ZUR VERMESSUNG EINES DENTALEN OBJEKTS**
METHOD FOR MEASURING A DENTAL OBJECT
PROCÉDÉ DE MESURE D'UN OBJET DENTAIRE

(30) Priorität: 14.08.2012 DE 102012214473
(43) Veröffentlichungstag der Anmeldung: 24.06.2015
(73) Patentinhaber: Sirona Dental Systems GmbH, 64625 Bensheim (DE)
(72) Erfinder: ADAMSON, Anders, 64297 Darmstadt (DE)
(74) Vertreter: Özer, Alpdeniz
(86) Internationale Anmeldenummer: PCT/EP2013/066994
(87) Internationale Veröffentlichungsnummer: WO 2014/027025

(56) Entgegenhaltungen:
- EP-A1- 1 434 028
- DE-A1-102007 005 625
- DE-A1-102008 054 985

## Beschreibung

### Technisches Gebiet

Die Erfindung betrifft ein Verfahren zur Vermessung eines dentalen Objekts mittels einer dentalen Kamera, wobei während der Vermessung mehrere optische dreidimensionale Aufnahmen des Objekts erzeugt werden.

### Stand der Technik

Aus dem Stand der Technik sind mehrere Verfahren und dentale Kameras zur optischen Vermessung eins dentalen Objekts bekannt.

In DE 10 2006 009 177 A1 ist ein dentales Handstück, insbesondere ein Kamera-Handstück, offenbart. Dieses Dokument zeigt auch ein Verfahren gemäß der Präambel des Anspruchs 1. Beim Betätigen einer Piezofolie wird eine Aufnahme durchgeführt. Das Auslösen einer Aufnahme kann durch ein akustisches Signal mittels einer akustischen Anzeigeeinrichtung erfolgen. Genauere Details zu der Ausgestaltung der akustischen Anzeigeeinrichtung sowie zu der Art des akustischen Signals sind nicht offenbart.

Darüber hinaus offenbart DE 10 2008 024 817 A1 ein Kamerahandstück, das beim Auslösen einer Aufnahme einen taktilen Alarm für den Benutzer erzeugt. Der taktile Alarm wird mittels einer taktilen Anzeige-Einrichtung erzeugt, die einen Piezoschwinger oder einen magnetischen Schwinger umfasst.Eine Rückmeldung über den Status der Vermessung bzw. über bestimmte Aufnahmebedingungen, wie eine ausreichende Fokussierung, oder eine erfolgreiche Registrierung, kann der Benutzer lediglich über eine visuelle Anzeigevorrichtung, wie mittels eines Monitors, erhalten.

Ein Nachteil der genannten dentalen Kameras und der Verfahren besteht also darin, dass der Benutzer während der Vermessung des dentalen Objekts immer wieder auf einen Monitor blicken muss, um eine Rückmeldung über den aktuellen Status oder über die Aufnahmebedingungen zu erlangen. Dies kann zu einer längeren Vermessungsdauer führen.

DE102008054985A1 offenbart ein Verfahren und eine Vorrichtung zur optischen Vermessung von dreidimensionalen Objekten mittels einer handgehaltenen, dentalen 3D-Kamera unter Verwendung eines Triangulationsverfahrens. Auch dieses Dokument zeigt ein Verfahren gemäß der Präambel des Anspruchs 1.

DE102007005625A1 offenbart eine Dentalkamera zur 3D-Vermessung von intraoralen Objekten mit optischen Mitteln. EP1434028A1 offenbart eine Vorrichtung und Verfahren zur Erzeugung eines kombinierten Bildes aus Teilbildern.

Die Aufgabe der vorliegenden Erfindung besteht also darin, ein Verfahren zur Vermessung bereitzustellen, dass eine einfache und sichere Überwachung des Vermessungsprozesses erlaubt.

### Darstellung der Erfindung

Diese Aufgabe wird durch das Verfahren in Anspruch 1 gelöst.

Ausführungsformen der Erfindung betreffen ein Verfahren zur Vermessung eines dentalen Objekts mittels einer dentalen Kamera, wobei während der Vermessung mehrere optische dreidimensionale Aufnahmen des Objekts erzeugt werden. Während der Vermessung wird ein akustischer Klang mittels eines Klanggebers erzeugt. Der Klang dient für den Benutzer als eine Rückkopplung und übermittelt dem Benutzer Informationen über einen aktuellen Status einer Registrierung der Aufnahmen und/oder über bestimmte Aufnahmebedingungen der dentalen Kamera. Während der optischen Vermessung werden mehrere dreidimensionale optische Aufnahmen des dentalen Objekts aufgenommen und anschließend zu einer Gesamtaufnahme zusammengefügt. Nach jeder einzelnen Aufnahme wird unter Verwendung eines Computers automatisch überprüft, ob ein Überlappungsbereich zwischen den zusammenzufügenden Aufnahmen bestimmte Registrierungsbedingungen für eine fehlerfreie Registrierung erfüllt. Der Klang wird mittels des Klanggebers erzeugt, falls die Registrierungsbedingungen für die überprüfte Aufnahme erfüllt sind. Alternativ kann der Klang erzeugt werden, falls die Registrierungsbedingungen für die überprüfte Aufnahme nicht mehr erfüllt sind. Die Registrierungsbedingungen sind eine ausreichende Größe des Überlappungsbereichs, eine ausreichende Welligkeit der Objektoberfläche im Überlappungsbereich, eine ausreichende Rauheit der Oberfläche im Überlappungsbereich, eine ausreichende Anzahl von charakteristischen Geometrien im Überlappungsbereich, eine ausreichende Anzahl von Messpunkten im Überlappungsbereich und/oder eine ausreichende Aufnahmequalität der Aufnahme im Überlappungsbereich.

Als Registrierungsverfahren kann beispielsweise das ICP-Registrierungsverfahren (Iterative-Closest-Point-Algorithmus) verwendet werden. Dieser Algorithmus ist ein bekanntes Verfahren zur Registrierung von zweidimensionalen bzw. dreidimensionalen Objekten. Das Ziel dieses Verfahrens ist es zwei unterschiedliche 3D-Modelle eines Objekts annäherungsweise genau aufeinander abzubilden. Dazu werden unterschiedliche Rotationen und Translationen auf korrespondierende Punktepaare der beiden 3D-Modelle angewendet. Dabei wird ein quadratischer Fehler der Abstände zwischen den Punktepaaren minimiert. Im Detail werden im ersten Schritt die nächsten Nachbarn eines bestimmten Punktes ermittelt. Im nächsten Schritt wird die Transformation zur Registrierung berechnet. Daraufhin wird die errechnete Transformation auf die zu registrierenden Punktepaare angewendet. Diese iterative Annäherung wird solange ausgeführt, bis die beiden 3D-Modelle im Überlagerungsbereich übereinstimmen.

Alternativ oder ergänzend dazu kann die Registrierung auch aufgrund der Farbe des aufgenommenen Objekts, der Oberflächenkrümmung des aufgenommenen Objekts oder aufgrund von geometrischen Übereinstimmungen erfolgen. Bei der Registrierung aufgrund geometrischer Übereinstimmungen wird ein Mustererkennungsalgorithmus verwendet, bei dem die zweite Aufnahme nach einem bestimmten geometrischen Muster, wie nach einer Okklusionsfläche eines bestimmten Zahns, aus der ersten Aufnahme durchsucht wird.

Die Registrierungsbedingungen werden beispielsweise dann nicht erfüllt, wenn die dentale Kamera in Relation zum Objekt zu schnell bewegt wird und dadurch die Größe des Überlappungsbereichs nicht ausreichend ist. Ein weiterer Grund könnte sein, dass ein Autofokus der dentalen Kamera unscharf eingestellt ist und dadurch das Objekt undeutlich abgebildet wird, so dass die Aufnahmequalität der jeweiligen Aufnahme nicht ausreichend ist. Ein weiterer Grund könnte sein, dass bewegliche Objekte wie die Zunge des Patienten oder Finger des behandelnden Zahnarztes, während der Vermessung erfasst werden. Dies führt dazu, dass die Überlappungsbereiche der Aufnahme nicht übereinstimmen. Ein weiterer Grund für eine fehlerhafte Registrierung könnte sein, dass die Optik der dentalen Kamera wegen der hohen Luftfeuchtigkeit im Mundraum eines Patienten beschlägt und dadurch die Aufnahmequalität verschlechtert wird. Die genannten Gründe könnten also dazu führen, dass die vorher festgelegten Registrierungsbedingungen nicht erfüllt sind. In solch einem Fall kann dann der Klang als ein Signalton erzeugt werden, um den Benutzer auf eine fehlerhafte Registrierung hinzuweisen. Alternativ dazu kann auch bei jeder erfolgreichen Registrierung ein bestimmter Klang erzeugt werden. Bei einer weiteren Alternative kann bei einer erfolgreichen Registrierung ein erster Klang und bei einer fehlerhaften Registrierung ein zweiter Klang, der sich vom ersten Klang deutlich unterscheidet, erzeugt werden.

Dadurch wird eine fehlerhafte Registrierung zwischen den zusammenzufügenden Aufnahmen verhindert. Durch die ausreichende Helligkeit und Rauheit der Oberfläche des Überlappungsbereichs wird im Gegensatz zu einer ebenen Fläche eine zuverlässige Registrierung ermöglicht. Die ausreichende Anzahl und Anordnung der charakteristischen Geometrien, wie beispielsweise von Fissuren oder Zahnhöckern, ermöglicht ebenfalls eine zuverlässige Registrierung. Bei einer ausreichenden Aufnahmequalität wird das dentale Objekt scharf und kontrastreich abgebildet. Ein Grund für geringen Kontrast könnte beispielsweise eine unzureichende Beleuchtung des Objekts sein. Ein Grund für die unscharfe Aufnahme könnte beispielsweise ein fehlerhaft eingestellter Autofokus sein. Eine ausreichende Anzahl von Messpunkten im Überlappungsbereich ist für eine erfolgreiche Mission ebenfalls erforderlich. Also erst wenn ein festgelegter Grenzwert für die Anzahl der Messpunkte im Übergangsbereich unterschritten wird, wird der Klang als ein Signalton für eine fehlerhafte Registrierung erzeugt.

Die optischen Aufnahmen werden mittels der dentalen Kamera vermessen, die beispielsweise nach einem Streifenprojektionsverfahren funktionieren kann. Bei einem Streifenprojektionsverfahren wird ein Muster aus mehreren parallelen Streifen auf das zu vermessende Objekt projiziert und anhand der Verzerrung dieser Streifen unter Verwendung eines Triangulationsverfahrens eine dreidimensionale Aufnahme des Objekts erzeugt.

Alternativ kann die optische Vermessung mittels einer dentalen Kamera erfolgen, die auf einem konfokalen optischen Verfahren oder auf einem Farbstreifenprojektionsverfahren beruht.

Bei dem Farbstreifenprojektionsverfahren wird ein Muster aus mehreren Farbstreifen auf das Objekt projiziert. Anschließend werden die Tiefenkoordinaten für die Messpunkte ermittelt und ein 3D-Modell des Objekts erzeugt. Die Farbstreifen können anhand ihrer Farbe eindeutig identifiziert werden. Für die farbliche Kodierung der Farbstreifen können beispielsweise vier Farbstreifen beziehungsweise drei Farbübergänge verwendet werden. Die Farbstreifen können beispielsweise mittels eines Dias erzeugt werden.

Die Streifenbreite für solche Streifenprojektionsverfahren kann beispielsweise 130 µm im Messvolumen am zu vermessendem Objekt betragen.

Zur optischen Vermessung kann auch ein anderes Streifenprojektionsverfahren verwendet werden, bei dem die Kodierung der Streifen unter Verwendung unterschiedlicher Lichteigenschaften, wie Intensität, Farbe, Polarisation, Kohärenz, Phase, Kontrast, Ort oder Laufzeit, erfolgt.

Zur Vermessung kann auch ein sogenanntes konfokales chromatisches Triangulationsverfahren, verwendet werden, bei dem die Konzepte einer konfokalen Vermessung und eines Triangulationsverfahrens miteinander kombiniert werden. Die grundlegende Idee besteht darin, dass die Oberfläche eines Objekts so eingefärbt wird, dass von einer Farbe direkt auf eine Höhenkoordinate geschlossen werden kann. Die Farben werden durch eine spektrale Aufspaltung des projizierten Lichtes erzeugt, wobei jede Wellenlänge auf eine eigene Höhenkoordinate fokussiert wird.

Während der Vermessung wird die handgehaltene dentale Kamera relativ zum dentalen Objekt, wie einen Unterkiefer oder einen Oberkiefer, bewegt, wobei in regelmäßigen Zeitabständen die dreidimensionalen optischen Aufnahmen erzeugt werden. Die einzelnen Aufnahmen können beispielsweise mit einer Taktfrequenz zwischen 10 Hz und 20 Hz erzeugt werden. Anschließend können die einzelnen Aufnahmen mittels eines Computers registriert und zu einer Gesamtaufnahme zusammengefügt werden.

Der Klanggeber kann ein Lautsprecher sein, der in die Dentalkamera integriert ist. Der erzeugte akustische Klang kann in seiner Dauer, in seiner Tonhöhe und/oder seiner Lautstärke beliebig gestaltet sein. Der Klang kann beispielsweise als ein Signalton für eine Überschreitung von festgelegten Aufnahmebedingungen, wie für einen zu schlechten Kontrast oder für eine zu geringe Schärfe der jeweiligen Aufnahme, gestaltet sein. Der Klang kann auch ein Signalton für eine fehlerhafte Registrierung der zusammenzufügenden Aufnahmen sein.

Ein Vorteil dieses Verfahrens besteht darin, dass der Benutzer unmittelbar akustisch über den erzeugten Klang Informationen über den aktuellen Status der Registrierung und/oder über bestimmte Aufnahmebedingungen der dentalen Kamera erhält. Dadurch ist es für den Benutzer, wie für einen Zahnarzt, nicht mehr notwendig während der Vermessung mehrmals auf einen Monitor zu blicken, um bestimmte Aufnahmebedingungen oder den Status der Registrierung zu überwachen.

Vorteilhafterweise kann ein Objektabstand des zu vermessenden Objekts relativ zu der dentalen Kamera ermittelt werden, wobei der Klang abhängig von diesem Objektabstand erzeugt wird.

Der Objektabstand kann anhand der dreidimensionalen Aufnahmen ermittelt werden. Beim Erzeugen der dreidimensionalen Aufnahmen werden mehrere Messpunkte auf der Objektoberfläche erfasst und die 3D-Koordinaten berechnet. Damit ist also auch der Abstand zwischen der Kamera und den jeweiligen Messpunkten bekannt. Bei der Ermittlung des Objektabstands werden die Abstände der jeweiligen Messpunkte innerhalb eines festgelegten Objektabschnitts gemittelt. Beispielsweise können die Abstände nur für diejenigen Messpunkte gemittelt werden, die auf einer definierten Okklusionsfläche eines bestimmten Zahns liegen.

Als eine Weiterbildung kann eine Okklusionsfläche auch in mehrere Sektoren unterteilt werden, wobei für jeden Sektor ein Schwerpunkt gebildet wird und lediglich die Abstände zwischen der Kamera und der jeweiligen Schwerpunkte der Sektoren gemittelt werden. Der Objektabstand kann also unmittelbar für jede einzelne dreidimensionale Aufnahme berechnet werden.

Alternativ dazu kann die Abstandsmesseinheit beispielsweise unter Verwendung eines Ultraschall-Laufzeitverfahrens, auch Sonar-Verfahren genannt, den Abstand der dentalen Kamera zum Objekt vermessen. Dabei wird ein Ultraschall von der Abstandmesseinheit erzeugt, der vom Objekt zurückgeworfen wird und mittels eines Sensors in der Abstandmesseinheit vermessen wird. Anhand der gemessenen Laufzeit des Ultraschalls kann dann der Abstand zum Objekt berechnet werden. Der Vorteil dieses Verfahrens besteht darin, dass der Abstand sehr schnell gemessen werden kann, ohne dass eine Probefokussierung oder eine Kontrastmessung notwendig ist.

Vorteilhafterweise kann anhand des Objektabstands ein Fokusabstand zwischen einem Schwerpunkt einer Objektoberfläche des zu vermessenden Objekts und einer Fokusebene der dentalen Kamera bestimmt werden, wobei der Klang abhängig von diesem Fokusabstand erzeugt wird.

Bei den verwendeten Streifenprojektionsverfahren und konfokalen Verfahren wird das ausgestrahlte Licht auf eine bestimmte Fokusebene beziehungsweise auf eine scharfe Schicht fokussiert, die durch die Einstellungen der Fokussieroptik in der dentalen Kamera festgelegt ist. Der Abstand zwischen der Fokusebene und der dentalen Kamera ist also bekannt und kann nach einem Lochkameramodell berechnet werden. Daher kann der Fokusabstand zwischen der Fokusebene und dem Schwerpunkt der Objektoberfläche berechnet werden, indem der Objektabstand vom Abstand der Fokusebene relativ zur dentalen Kamera subtrahiert wird.

Innerhalb eines Messfeldes kann der Abstand zum Fokusebene geringfügig variieren, so dass als relevante Messgröße für die Klangerzeugung der mittlere Abstand zu Fokusebene innerhalb des Messfeldes verwendet werden kann.

Vorteilhafterweise kann der erzeugte Klang ein modulierter Klang sein und abhängig von Fokusabstand zwischen der Objektoberfläche und der Fokusebene der dentalen Kamera in seiner Tonhöhe und/oder seiner Lautstärke verändert werden.

Der modulierte Klang könnte beispielsweise ein dissonanter Ton sein, der den Fokusabstand über die Tonhöhe und/oder über die Lautstärke anzeigt.

Vorteilhafterweise kann der Klang ein kurzer Klick-Klang sein, der erzeugt wird, sobald festgelegte Grenzen eines scharfen Fokusbereiches der dentalen Kamera überschritten werden.

Die Grenzen eines scharfen Fokusbereiches können beispielsweise +5 mm und -5 mm relativ zur Fokusebene betragen.

Ein weiteres Verfahren der Erfindung betrifft ein Verfahren zur Vermessung eines dentalen Objekts mittels einer dentalen Kamera, wobei während der Vermessung mehrere optische dreidimensionale Aufnahmen des Objekts erzeugt werden. Während der Vermessung wird ein akustischer Klang mittels eines Klanggebers erzeugt. Der Klang dient für den Benutzer als eine Rückkopplung und übermittelt dem Benutzer Informationen über einen aktuellen Status einer Registrierung der Aufnahmen und/oder über bestimmte Aufnahmebedingungen der dentalen Kamera. Eine Bewegungsgeschwindigkeit der dentalen Kamera relativ zum Objekt wird mittels eines Bewegungssensors an der dentalen Kamera oder durch Auswertung der vermessenen Aufnahmen ermittelt, wobei der Klang abhängig von der ermittelten Bewegungsgeschwindigkeit erzeugt wird.

Die optischen Aufnahmen werden mittels der dentalen Kamera vermessen, die beispielsweise nach einem Streifenprojektionsverfahren funktionieren kann. Bei einem Streifenprojektionsverfahren wird ein Muster aus mehreren parallelen Streifen auf das zu vermessende Objekt projiziert und anhand der Verzerrung dieser Streifen unter Verwendung eines Triangulationsverfahrens eine dreidimensionale Aufnahme des Objekts erzeugt.

Alternativ kann die optische Vermessung mittels einer dentalen Kamera erfolgen, die auf einem konfokalen optischen Verfahren oder auf einem Farbstreifenprojektionsverfahren beruht.

Bei dem Farbstreifenprojektionsverfahren wird ein Muster aus mehreren Farbstreifen auf das Objekt projiziert. Anschließend werden die Tiefenkoordinaten für die Messpunkte ermittelt und ein 3D-Modell des Objekts erzeugt. Die Farbstreifen können anhand ihrer Farbe eindeutig identifiziert werden. Für die farbliche Kodierung der Farbstreifen können beispielsweise vier Farbstreifen beziehungsweise drei Farbübergänge verwendet werden. Die Farbstreifen können beispielsweise mittels eines Dias erzeugt werden.

Die Streifenbreite für solche Streifenprojektionsverfahren kann beispielsweise 130 µm im Messvolumen am zu vermessenden Objekt betragen.

Zur optischen Vermessung kann auch ein anderes Streifenprojektionsverfahren verwendet werden, bei dem die Kodierung der Streifen unter Verwendung unterschiedlicher Lichteigenschaften, wie Intensität, Farbe, Polarisation, Kohärenz, Phase, Kontrast, Ort oder Laufzeit, erfolgt.

Zur Vermessung kann auch ein sogenanntes konfokales chromatisches Triangulationsverfahren, verwendet werden, bei dem die Konzepte einer konfokalen Vermessung und eines Triangulationsverfahrens miteinander kombiniert werden. Die grundlegende Idee besteht darin, dass die Oberfläche eines Objekts so eingefärbt wird, dass von einer Farbe direkt auf eine Höhenkoordinate geschlossen werden kann. Die Farben werden durch eine spektrale Aufspaltung des projizierten Lichtes erzeugt, wobei jede Wellenlänge auf eine eigene Höhenkoordinate fokussiert wird.

Während der Vermessung wird die handgehaltene dentale Kamera relativ zum dentalen Objekt, wie einen Unterkiefer oder einen Oberkiefer, bewegt, wobei in regelmäßigen Zeitabständen die dreidimensionalen optischen Aufnahmen erzeugt werden. Die einzelnen Aufnahmen können beispielsweise mit einer Taktfrequenz zwischen 10 Hz und 20 Hz erzeugt werden. Anschließend können die einzelnen Aufnahmen mittels eines Computers registriert und zu einer Gesamtaufnahme zusammengefügt werden.

Der Klanggeber kann ein Lautsprecher sein, der in die Dentalkamera integriert ist. Der erzeugte akustische Klang kann in seiner Dauer, in seiner Tonhöhe und/oder seiner Lautstärke beliebig gestaltet sein. Der Klang kann beispielsweise als ein Signalton für eine Überschreitung von festgelegten Aufnahmebedingungen, wie für einen zu schlechten Kontrast oder für eine zu geringe Schärfe der jeweiligen Aufnahme, gestaltet sein. Der Klang kann auch ein Signalton für eine fehlerhafte Registrierung der zusammenzufügenden Aufnahmen sein.

Der Bewegungssensor kann in die dentale Kamera integriert sein und die relative Bewegungsgeschwindigkeit der dentalen Kamera relativ zum Objekt bestimmen. Die relative Bewegungsgeschwindigkeit kann aber auch durch Auswertung der vermessenen Aufnahmen ermittelt werden, wobei die Bewegungsgeschwindigkeit anhand eines Versatzes des Objekts in aufeinanderfolgenden Aufnahmen und anhand des bekannten Zeitabstands zwischen den Aufnahmen bestimmt wird.

Alternativ dazu kann die Bewegungsgeschwindigkeit durch Auswertung der vermessenen Aufnahmen ermittelt werden. Dabei kann beispielsweise das ICP-Verfahren verwendet werden. Dabei werden relative Transformationen des vermessenen Objekts in den nacheinander aufgenommenen einzelnen Aufnahmen berechnet. Diese Transformationen können Verschiebungen und Rotationen des Objekts darstellen. In Kenntnis der Aufnahmefrequenz und der ermittelten Transformationen kann dann die Bewegungsgeschwindigkeit für einzelne Messpunkte des Objekts ermittelt werden. Als Messgröße für die Klangerzeugung kann eine durchschnittliche Bewegungsgeschwindigkeit innerhalb eines Messfeldes verwendet werden. Dadurch werden also die bei einer Rotation der Kamera entstehenden Bewegungsgeschwindigkeiten der einzelnen Messpunkte innerhalb des Messfeldes herausgemittelt. Das Messfeld kann beispielsweise eine Größe von 17 mm x 14 mm betragen.

Ein Vorteil dieses Verfahrens besteht darin, dass der Benutzer unmittelbar akustisch über den erzeugten Klang Informationen über den aktuellen Status der Registrierung und/oder über bestimmte Aufnahmebedingungen der dentalen Kamera erhält. Dadurch ist es für den Benutzer, wie für einen Zahnarzt, nicht mehr notwendig während der Vermessung mehrmals auf einen Monitor zu blicken, um bestimmte Aufnahmebedingungen oder den Status der Registrierung zu überwachen.

Vorteilhafterweise kann der Klanggeber einen modulierten Klang abgeben, wobei die Tonhöhe und/ oder die Lautstärke des Klangs abhängig von der Bewegungsgeschwindigkeit der dentalen Kamera relativ zum Objekt verändert werden kann.

Auf diese Art und Weise gibt der modulierte Klang über die Tonhöhe und/oder über die Lautstärke die relative Bewegungsgeschwindigkeit an.

Vorteilhafterweise kann der erzeugte Klang ein kurzer Klick-Klang sein, der erzeugt wird, sobald eine festgelegte kritische Bewegungsgeschwindigkeit überschritten wird.

Dadurch wird der Benutzer mittels eines solchen Signaltons auf eine Überschreitung der kritischen Bewegungsgeschwindigkeit hingewiesen.

Die kritische Bewegungsgeschwindigkeit kann beispielsweise 2 cm/Sekunde betragen.

### Kurzbeschreibung der Zeichnungen

Die Erfindung wird anhand der Zeichnungen erläutert. Es zeigt, die
- Fig. 1: eine Skizze zur Verdeutlichung des vorliegenden Verfahrens zur Vermessung eines dentalen Objekts, die
- Fig. 2: eine Skizze zur Verdeutlichung einer Klangerzeugung abhängig von einem Objektabstand, die
- Fig. 3: eine Skizze zur Verdeutlichung einer alternativen Ausführungsform für die Bestimmung des Objektabstandes, die
- Fig. 4: ein Diagramm zur Verdeutlichung der Erzeugung eines modulierten Klangs in Abhängigkeit vom Objektabstand, die
- Fig. 5: eine Skizze zur Verdeutlichung der Bestimmung der Bewegungsgeschwindigkeit anhand der Aufnahmen.

### Ausführungsbeispiel

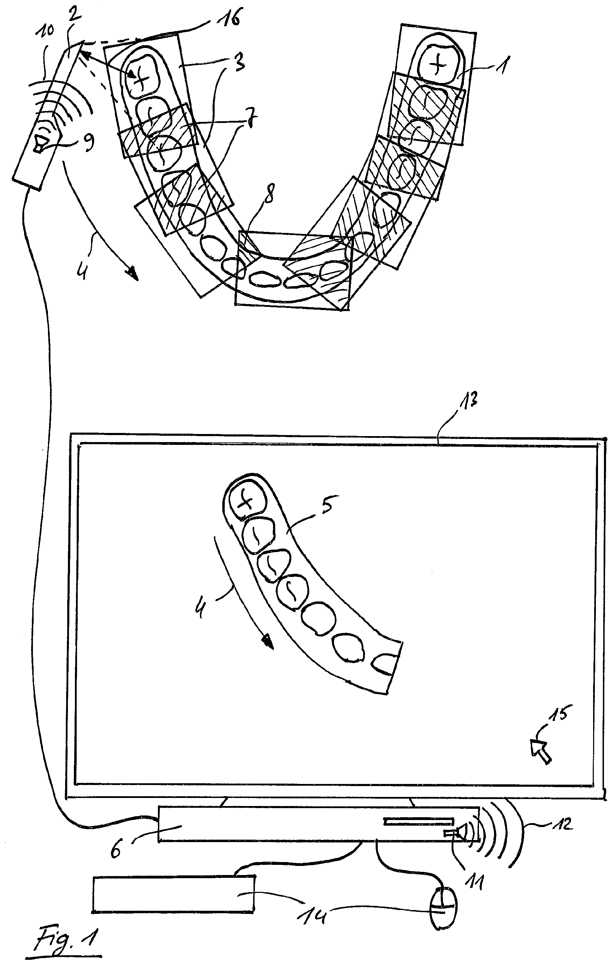

Die Fig. 1 zeigt eine Skizze zur Verdeutlichung des vorliegenden Verfahrens zur Vermessung eines dentalen Objekts 1, die eines Unterkiefers, mittels einer dentalen Kamera 2, wobei während der Vermessung mehrere einzelne optische dreidimensionale Aufnahmen 3 erzeugt werden. Die dreidimensionalen Aufnahmen 3, die in Form von Rechtecken dargestellt sind, werden mittels der dentalen Kamera 2 vermessen, die entlang eines Vermessungswegs 4 relativ zu Objekt 1 bewegt wird. Die dentale Kamera 2 kann beispielsweise eine handgehaltene Kamera sein, die das Objekt 1 unter Verwendung eines Streifenprojektionsverfahrens vermisst. Dabei können die Streifen unter Verwendung unterschiedlicher Lichteigenschaften, wie Intensität, Farbe, Polarisation, Kohärenz, Phase, Kontrast, Ort und Laufzeit, kodiert sein. Als Vermessungsverfahrens kann auch ein konfokales Vermessungsverfahren verwendet werden.

Die einzelnen Aufnahmen 3 werden mittels eines Registrierungsverfahrens zu einer Gesamtaufnahme 5 zusammengefügt, wobei mittels eines Computers 6 automatisch überprüft wird, ob die Überlappungsbereiche 7, die gestrichelt dargestellt sind, bestimmte Registrierungsbedingungen für eine fehlerfreie Registrierung erfüllen. Die Registrierungsbedingungen können eine ausreichende Größe der Überlappungsbereiche 7, eine ausreichende Welligkeit der Objektoberflächenüberlappungsbereich, eine ausreichende Rauheit des Oberflächenüberlappungsbereich, eine ausreichende Anzahlung von charakteristischen Geometrien im Überlappungsbereich 7, eine ausreichende Anzahl von Messpunkten im Überlappungsbereich 7 und/oder eine ausreichende Aufnahmequalität der Aufnahme 3 im Überlappungsbereich 7 sein. Der dritte Überlappungsbereich 8 erfüllt diese Registrierungsbedingungen nicht, da die Größe dieses Überlappungsbereichs 8 zu gering für eine erfolgreiche Registrierung ist. In solch einem Fall wird mittels eines Klanggebers 9, der in der Dentalkamera 2 angeordnet ist, ein charakteristischer Klang 10, der durch die radialen Linien dargestellt ist, als Rückkopplung für den Benutzer erzeugt. Alternativ dazu ein zweiter Klang erzeugt werden, falls die Registrierungsbedingungen erfüllt werden. Alternativ oder ergänzend zum ersten Klanggeber 9 kann ein zweiter Klanggeber 11 außerhalb der Dentalkamera 2 beispielsweise innerhalb des Computers 6 angeordnet sein, der einen zweiten charakteristischen Klang 12 erzeugt. Die Klänge 10, 12 können beliebig ausgestaltet sein. Der Klang kann beispielsweise ein charakteristischer Klick-Klang oder ein in seiner Tonhöhe bzw. in seiner Lautstärke modulierter Klang sein. Während der Vermessung können weitere einzelne Aufnahmen 3 zur Gesamtaufnahme 5 registriert werden bis das gesamte dentale Objekt 1 vermessen wurde. Die Gesamtaufnahme 5 kann beispielsweise mittels einer Anzeigevorrichtung 13, wie mittels eines Monitors, angezeigt werden, wobei an dem Computer 6 Eingabemittel 14, wie eine Maus und eine Tastatur, angeschlossen sein können. Der Benutzer kann dann mittels der Eingabemittel 14 über einen Cursor 15 die dreidimensionale Gesamtaufnahme 5 drehen und verschieben, um die Blickrichtung zu verändern.

Alternativ dazu kann der Klang abhängig von einem Objektabstand 16 zwischen der Dentalkamera 2 und dem zu vermessenden Objekt 1 erzeugt werden.

Bei einer weiteren Alternative kann der Klang abhängig von einer Bewegungsgeschwindigkeit der dentalen Kamera 2 entlang des Vermessungswegs 4 relativ zum Objekt 1 erzeugt werden.

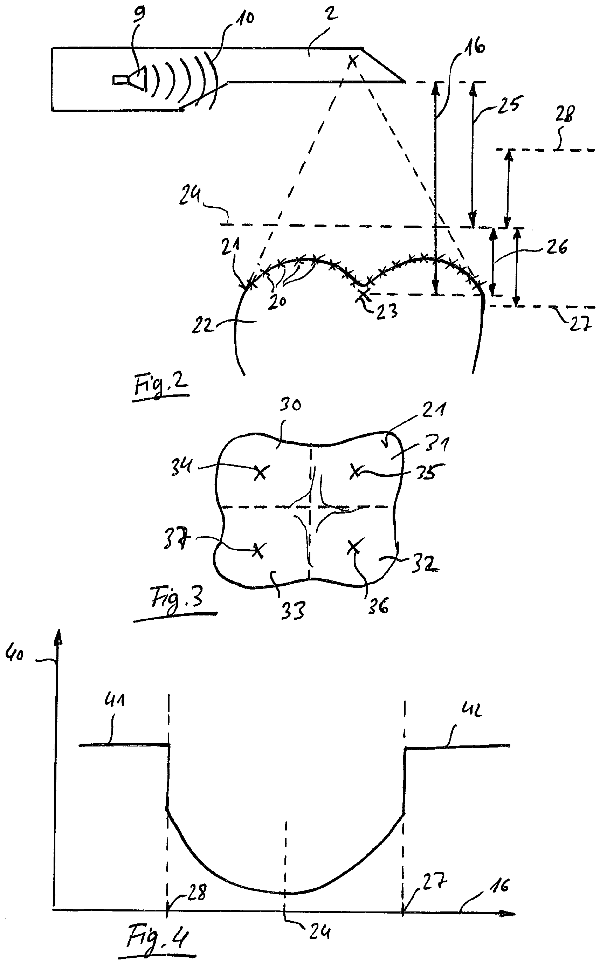

Die Fig. 2 zeigt eine Skizze zur Verdeutlichung eines charakteristischen Klangs 10 mittels des Klanggebers 9, der in die dentale Kamera 2 integriert ist, abhängig vom Objektabstand 16. Im dargestellten Fall wird der Objektabstand 16 bestimmt, indem die einzelnen Abstände der Messpunkte 20 auf einer festgelegten Okklusalfläche 21 eines bestimmten Backenzahns 22 relativ zur Dentalkamera 2 gemittelt werden und dadurch ein Schwerpunkt 23 berechnet wird, der durch ein großes x dargestellt ist. Der Objektabstand 16 ist also der gemittelte Abstand zwischen der Dentalkamera 2 und der relevanten Objektoberfläche, wie einer Okklusalfläche 21. Der Objektabstand kann auch durch Mittelung über alle Messpunkte innerhalb eines Messfeldes berechnet werden. Eine Fokusebene 24 weist einen bekannten Abstand 25 zur Dentalkamera 2 auf, wobei dieser Abstand durch das Lochkameramodell unter Berücksichtigung der verwendeten Optik berechnet werden kann. Bei einem Streifenprojektionsverfahren werden die produzierten Streifen in der Fokusebene scharf abgebildet. In Kenntnis des bestimmten Objektabstandes 16 und des bekannten Abstandes 25 zur Fokusebene kann also ein Fokusabstand 26 durch Subtraktion berechnet werden. Falls dieser Fokusabstand 26 eine festgelegte untere Grenze 27 oder eine obere festgelegte Grenze 28 überschreitet wird als Signalton der Klang 10 erzeugt. Die festgelegten Grenzen können beispielsweise +- 5 mm sein. Der erzeugte Klang 10 kann auch ein modulierter Klang sein, der abhängig vom Fokusabstand 26 in seine Tonhöhe und/oder Lautstärke moduliert wird.

Die Fig. 3 zeigt eine Skizze zur Verdeutlichung einer alternativen Ausführungsform für die Bestimmung des Objektabstandes 16 aus Fig. 2. Dabei wird die relevante Okklusionsfläche 21 in vier Sektoren 30, 31, 32 und 33 unterteilt, wobei für jeden der Sektoren ein erster Schwerpunkt 34, ein zweiter Schwerpunkt 35, ein dritter Schwerpunkt 36 und ein vierter Schwerpunkt 37 bestimmt wird. Anschließend werden die Abstände der jeweiligen Schwerpunkte 34, 35, 36 und 37 relativ zur Kamera 2 gemittelt und dadurch ein Schwerpunkt der gesamten Okklusalfläche 21 bestimmt.

Die Fig. 4 zeigt eine Skizze zur Verdeutlichung der Erzeugung eines modulierten Klangs, wobei der dargestellte Graph die Tonhöhe bzw. die Frequenz des erzeugten Klangs 40 in Abhängigkeit vom Objektabstand 16 darstellt. In der Fokusebene 24 ist die Tonhöhe des Klangs am tiefsten und steigt bis zu den festgelegten Grenzen 27 und 28 langsam an. Beim beschreiten der Grenzen 27 oder 28 steigt die Tonhöhe 40 in den Bereichen 41 und 42 sprunghaft an, so dass der Zahnarzt deutlich erkennen kann, dass die festgelegten Grenzen 27 oder 28 überschritten wurden.

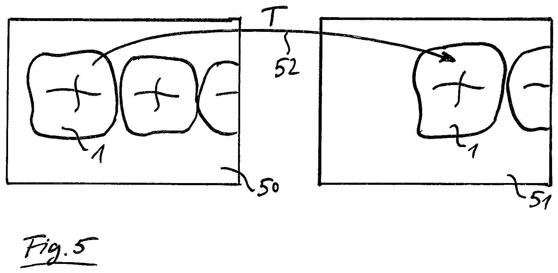

Die Fig. 5 zeigt eine Skizze zur Verdeutlichung der Bestimmung der Bewegungsgeschwindigkeit der Dentalkamera 2 relativ zum Objekt 1 aus Fig. 1 unter Verwendung eines ICP-Algorithmus. Dabei wird in einer ersten Aufnahme 50 ein Abschnitt des Objekts 1 aufgenommen die Dentalkamera 2 entlang des Vermessungswegs 4 aus Fig. 1 bewegt und eine darauffolgende zweite Aufnahme 51 durchgeführt, wobei der Abschnitt des Objekts 1 verschoben wurde. Unter Verwendung des ICP-Algorithmus wird eine Translation 52 die eine Kombination aus einer linearen Verschiebung und einer Rotation sein kann, ermittelt. Anschließend wird in Kenntnis der Aufnahmefrequenz und der ermittelten Translation 52 eine Bewegungsgeschwindigkeit für jeden der Messpunkte auf dem Objekt 1 ermittelt. Anschließend wird eine mittlere Bewegungsgeschwindigkeit durch Mittlung der Bewegungsgeschwindigkeit der einzelnen Messpunkte berechnet. Sobald eine kritische Bewegungsgeschwindigkeit überschritten wird, kann der Klang 10 aus Fig. 1 erzeugt werden, um dem Zahnarzt anzuzeigen, dass er die Dentalkamera 2 zu schnell bewegt.

### Bezugszeichen

- 1: Objekt
- 2: Kamera
- 3: Aufnahme
- 4: Vermessungsweg
- 5: Gesamtaufnahme
- 6: Computer
- 7: Überlappungsbereich
- 8: Überlappungsbereich
- 9: Klanggeber
- 10: Klang
- 11: Klanggeber
- 12: Klang
- 13: Gesamtaufnahme
- 14: Eingabemittel
- 15: Cursor
- 16: Objektabstand
- 20: Messpunkt
- 21: Okklusalfläche
- 22: Backenzahn
- 23: Schwerpunkt
- 24: Fokusebene
- 25: Abstand
- 26: Fokusabstand
- 27: Grenze
- 28: Grenze
- 30: Sektor
- 31: Sektor
- 32: Sektor
- 33: Sektor
- 34: Schwerpunkt
- 35: Schwerpunkt
- 36: Schwerpunkt
- 37: Schwerpunkt
- 40: Tonhöhe
- 41: Bereich
- 42: Bereich
- 50: Aufnahme
- 51: Aufnahme
- 52: Translation

## Patentansprüche

1. Verfahren zur Vermessung eines dentalen Objekts (1) mittels einer dentalen Kamera (2), wobei während der Vermessung mehrere optische dreidimensionale Aufnahmen (3, 50, 51) des Objekts (1) erzeugt werden, wobei während der Vermessung ein akustischer Klang (10, 12) mittels eines Klanggebers (9, 11) erzeugt wird, **dadurch gekennzeichnet, dass** der Klang (10, 12) als eine Rückkopplung für den Benutzer geeignet ist , wobei eine Bewegungsgeschwindigkeit der dentalen Kamera (2) relativ zum Objekt (1) mittels eines Bewegungssensors an der dentalen Kamera (2) oder durch Auswertung der vermessenen Aufnahmen (50, 51) ermittelt wird, wobei der Klang (10, 12) abhängig von der ermittelten Bewegungsgeschwindigkeit erzeugt wird.

## Claims

1. Method for measuring a dental object (1) using a dental camera (2), wherein several optical three-dimensional images (3, 50, 51) of the object (1) are produced during the measurement, wherein an acoustic sound (10, 12) is generated by a sound generator (9, 11) during the measurement, **characterised in that** the sound (10, 12) is suitable as feedback for the user, wherein the speed of movement of the dental camera (2) relative to the object (1) is determined using a motion sensor on the dental camera (2) or by evaluating the measured images (50, 51), wherein the sound (10, 12) is generated depending on the determined speed of movement.

## Revendications

1. Procédé de mesure d'un objet dentaire (1) au moyen d'une caméra dentaire (2), dans lequel pendant la mesure plusieurs prises de vue optiques tridimensionnelles (3, 50, 51) de l'objet (1) sont produites, dans lequel un son acoustique (10, 12) est produit pendant la mesure au moyen d'un générateur de son (9, 11), **caractérisé en ce que** le son (10, 12) convient à titre de rétroaction pour l'utilisateur, une vitesse de mouvement de la caméra dentaire (2) par rapport à l'objet (1) étant déterminée au moyen d'un capteur de mouvement sur la caméra dentaire (2) ou par évaluation des prises de vue mesurées (50, 51), le son (10, 12) étant produit en fonction de la vitesse de mouvement déterminée.
